# FASCICULE DE BREVET EUROPEEN

(11) **EP 0 532 540 B1**
(45) Date de publication et mention de la délivrance du brevet: **28.12.1994**
(21) Numéro de dépôt: 91909876.4
(22) Date de dépôt: 04.06.1991
(51) Int. Cl.: C07C 19/08, C07C 17/00

(54) **PROCEDE DE PREPARATION DE 1,1-DICHLORO-1-FLUOROETHANE**
VERFAHREN ZUR HERSTELLUNG VON 1,1-DICHLOR-1-FLUORETHAN
METHOD FOR PREPARING 1,1-DICHLORO-1-FLUOROETHANE

(30) Priorité: 08.06.1990 BE 9000579
(43) Date de publication de la demande: 24.03.1993
(73) Titulaire: SOLVAY, B-1050 Bruxelles (BE)
(72) Inventeur: JANSSENS, Francine, B-1800 Vilvoorde (BE); FRANKLIN, James, B-1170 Bruxelles (BE)
(74) Mandataire: Marckx, Frieda
(86) Numéro de dépôt international: BE9100034
(87) Numéro de publication internationale: WO9118852

(56) Documents cités:
- EP-A- 0 361 578

## Description

La présente invention concerne un procédé pour la fabrication de 1,1-dichloro-1-fluoroéthane au départ de 1,1-dichloro-éthylène également appelé chlorure de vinylidène (VC₂), par réaction avec du fluorure d'hydrogène en milieu liquide en l'absence de catalyseur(s) et à température élevée.

Le 1,1-dichloro-1-fluoroéthane est un produit de synthèse, comportant dans sa molécule outre les atomes de chlore, de fluor et de carbone, des atomes d'hydrogène. Le point d'ébullition de ce composé est de 32°C sous pression atmosphérique. Il peut être utilisé seul notamment en tant qu'agent gonflant ou en mélange avec d'autres composés chlorofluorés hydrogénés ou non.

Les procédés "industriels" connus pour obtenir le 1,1-dichloro-1-fluoroéthane sont tous réalisés au départ de 1,1,1-trichloroéthane bien qu'il soit connu de longue date que l'on peut obtenir ce produit par hydrofluoration de chlorure de vinylidène.

Ainsi, le document Journal of Am. Chem. Soc. 1943, 65 p. 1272 décrit déjà que la réaction entre 1 mole de chlorure de vinylidène et 4 moles de fluorure d'hydrogène effectuée pendant trois heures à 65°C permet notamment d'obtenir 50 % de 1,1-dichloro-1-fluoroéthane, 5 % de 1,1,1-trichloroéthane et 15 % de goudrons.

Par ailleurs, le brevet britannique 627,773 divulgue notamment la réaction entre des températures de 10°C et 35°C pendant 1h45', de 8 moles de chlorure de vinylidène avec environ 8,7 moles de fluorure d'hydrogène en présence de chlorure d'étain, avec l'obtention d'un taux de conversion de 32,7 % en 1,1-dichloro-1-fluoroéthane calculé par rapport au chlorure de vinylidène mis en oeuvre.

De tels rendements et de telles puretés étaient toutefois trop peu élevés pour justifier une exploitation industrielle voire même une poursuite des études de la fabrication de 1,1-dichloro-1-fluoroéthane par hydrofluoration de chlorure de vinylidène et cette voie a par conséquent été abandonnée au profit de celle au départ de 1,1,1-trichloroéthane qui elle ne présentait apparemment pas les mêmes désavantages.

La demanderesse a maintenant trouvé un procédé pour fabriquer du 1,1-dichloro-1-fluoroéthane par hydrofluoration de chlorure de vinylidène qui ne présente plus les inconvénients des procédés mentionnés ci-dessus.

La présente invention concerne donc un procédé pour la fabrication du 1,1-dichloro-1-fluoroéthane par réaction du fluorure d'hydrogène avec le chlorure de vinylidène dans lequel on effectue la réaction dans un milieu liquide à une température supérieure à 70°c et en l'absence de catalyseur.

Le fluorure d'hydrogène est impérativement mis en oeuvre dans le présent procédé sous forme anhydre et avec une pureté supérieure à 95 % et de préférence supérieure à 99 % vol. Il peut être introduit dans le réacteur sous forme gazeuse ou liquide.

Le chlorure de vinylidène mis en oeuvre doit répondre aux mêmes spécifications que celles décrites ci-dessus pour le fluorure d'hydrogène c'est-à-dire qu'il doit être anhydre et pur.

Les quantités respectives de fluorure d'hydrogène et de chlorure de vinylidène introduites dans le réacteur sont sans grande importance en elles-mêmes. Par contre le rapport molaire dans lesquels ces deux réactifs sont introduits dans le réacteur, doit être impérativement compris entre 1,5 à 3 moles de HF pour 1 mole de chlorure de vinylidène, si l'on désire observer les résultats avantageux procurés par le procédé de l'invention.

Le mélange réactionnel à l'intérieur du réacteur d'hydrofluoration doit être maintenu à l'état liquide pour le bon déroulement du procédé de l'invention. Pour ce faire, on peut utiliser tout moyen connu. Un moyen pratique, ayant donné de bons résultats, consiste à opérer dans un réacteur maintenu sous pression.

La température de réaction est en général comprise entre 75°C et 130°C et de préférence entre 80°C et 125°C. Dans ces conditions, la pression dans le réacteur est en général comprise entre 2 et 30 bars et de préférence entre 5 et 25 bars.

La sélectivité en 1,1-dichloro-1-fluoroéthane obtenu dans le procédé de l'invention est très élevée et atteint, pour des conditions opératoires de 100°C et pour un rapport molaire initial VC₂/HF de 1/2, encore 85 % lorsque le taux de conversion du VC₂ est aussi élevé que 98,7 %.

La quantité d'oligomères et goudrons formés lorsque l'on opère selon le procédé de l'invention, qui constituent un obstacle à l'exploitation industrielle des procédés antérieurs connus, est très faible et concurrentielle avec ce qui est observé avec les procédés réalisés au départ de 1,1,1-trichloroéthane.

Ainsi, lorsque l'on opère à des températures supérieures à 70°C, on observe que la quantité totale d'oligomères est inférieure à 5 % mol de la quantité totale de chlorure de vinylidène mise en oeuvre. Il est même possible, en jouant sur les paramètres réactionnels, d'atteindre des quantités d'oligomères inférieures à 1 % mol, voire 0,2 % mol de la quantité de chlorure de vinylidène mise en oeuvre.

Bien que le procédé de l'invention soit réalisé de façon purement thermique en l'absence de tout catalyseur, avec des quantités de réactifs et de produits réactionnels tels qu'explicités ci-avant, il est clair que toute variante dans laquelle on utilise un procédé thermique mais dans lequel des réactifs en excès tels que le chlorure de vinylidène, le fluorure d'hydrogène ou des produits en excès issus de la réaction tels que le chlorure d'hydrogène ou le 1,1-difluoro-1-chloroéthane, sont introduits temporairement ou en continu en des quantités supérieures ou inférieures à celles spécifiées ci-avant, fait également partie de la présente invention.

Les réacteurs utilisés dans le procédé de l'invention peuvent être réalisés en différents matériaux tels que l'acier, l'acier inoxydable ou encore en alliages divers tels que le MONEL, l'INCONEL et l'HASTELLOY. On peut également utiliser des réacteurs dans lesquels la paroi intérieure est revêtue d'un revêtement inerte tel qu'une couche d'une résine inerte dans les conditions de réaction, telles que par exemple des résines fluorées.

Les réacteurs sont avantageusement équipés de dispositifs techniques permettant d'améliorer le contact entre les réactifs. On peut ainsi disposer dans les réacteurs des agitateurs, ou encore prévoir des dispositifs d'introduction des réactifs, permettant une dispersion efficace des réactifs au sein du mélange réactionnel.

Le mode d'introduction ainsi que les débits des réactifs à l'entrée des réacteurs sont réglés de façon à maintenir les proportions désirées des réactifs au sein du milieu réactionnel liquide. Ils sont dès lors fonction, notamment de la température, du taux de remplissage des réacteurs, du temps de séjour et des débits de soutirage appliqués.

Dans les conditions décrites ci-dessus, la réaction peut être conduite en discontinu on en continu. Si elle est conduite en discontinu, on opère la réaction habituellement dans des autoclaves agités et fermés et lorsqu'elle est conduite en continu, on opère de préférence dans des réacteurs de type mélangeur équipés de systèmes d'introduction des réactifs et de soutirage des produits en continu.

Quel que soit le mode de conduite de la réaction, en discontinu ou en continu, du procédé de l'invention, il est essentiel que les moyens de soutirage soient réglés de façon à permettre de soutirer en phase gazeuse essentiellement tout le chlorure d'hydrogène produit par le procédé de l'invention, les autres produits, issus de la réaction ou résiduaires, dont la température d'ébullition est supérieure à celle du chlorure d'hydrogène, étant soutirés en phase liquide.

Un des réactifs, ou les deux, peuvent être éventuellement introduits en un seul, ou en plusieurs points espacés des réacteurs. On peut ainsi prévoir notamment plusieurs points équipés de moyens d'introduction du chlorure de vinylidène dans les réacteurs, tout en respectant toutefois les rapports molaires de réactifs introduits.

La réaction peut être réalisée dans un réacteur unique ou dans plusieurs réacteurs montés en série. Dans ce cas, on peut également prévoir divers modes d'introduction des réactifs. Les deux réactifs peuvent être introduits dans un même réacteur ou, l'on peut répartir l'introduction d'un des réactifs dans chacun des réacteurs. On peut également utiliser plusieurs réacteurs, alimentés respectivement en chacun des réactifs, les mélanges formés dans chacun des réacteurs circulant dans le même sens ou à contre-courant.

Le mode de soutirage en phase gazeuse du chlorure d'hydrogène formé par la réaction peut être effectué par tout moyen connu à cet effet. Un moyen ayant donné de bons résultats consiste à équiper le réacteur d'une colonne de reflux. Selon l'efficacité du reflux appliqué, le chlorure d'hydrogène contient en outre des composés organiques légers sous-produits qui peuvent être éliminés, si on le désire, par des moyens habituels tels que la distillation fractionnée.

Les réacteurs sont également avantageusement équipés de moyens permettant de récupérer une partie de la phase liquide. Afin de favoriser la récupération en phase liquide, on opère de préférence avec un milieu réactionnel maintenu sous une pression d'environ 10 à 20 bars.

Enfin, le mélange liquide issu du réacteur peut ensuite être soumis à des opérations de séparation en une ou plusieurs étapes permettant de récupérer du 1,1-dichloro-1-fluoroéthane.

Certains des sous-produits et impuretés peuvent être aisément éliminés par distillation. C'est notamment le cas pour le 1-chloro-1,1-difluoroéthane et le 1,1,1-trifluoroéthane, ainsi que pour les oligomères formés en cours de réaction. Toutefois, le 1,1-dichloro-1-fluoroéthane contient généralement en tant qu'impuretés de petites quantités de composés insaturés chlorés ou chlorofluorés indésirables qui sont très difficilement séparables par distillation en raison de leur point d'ébullition très proche de celui du 1,1-dichloro-1-fluoroéthane. En effet, en plus du chlorure de vinylidène n'ayant pas réagi, les principales impuretés insaturées qui peuvent être présentes dans le 1,1-dichloro-1-fluoroéthane à purifier sont principalement le dichloracétylène, les 1,2-dichloro-1-fluoroéthylènes (isomères cis et trans), le 1,2-dichloroéthylène-trans ainsi que des traces de 1-chloro-1-fluoroéthylène.

Les produits insaturés chlorés tels que le chlorure de vinylidène, encore éventuellement présents dans le milieu réactionnel, peuvent être éliminés par toute méthode chimique ou physique connue pour ce faire. Dans le cas particulier du chlorure de vinylidène des méthodes ayant donné de bons résultats consistent dans la bromation ou la chloration de ce composé suivie d'une séparation par distillation du composé saturé obtenu.

Si l'on désire récupérer du 1,1-dichloro-1-fluoroéthane avec une pureté supérieure à 99 % volume, un procédé ayant donné de bons résultats consiste à :
. soumettre d'abord la phase liquide organique, issue du réacteur d'hydrofluoration et préalablement séparée de la phase liquide contenant les inorganiques c.à.d. essentiellement le fluorure d'hydrogène, à une distillation afin d'éliminer en phase vapeur les produits dont le point d'ébullition est inférieur à celui du 1,1-dichloro-1-fluoroéthane c.à.d. essentiellement pour éliminer les traces de 1-chloro-1,1-difluoroéthane et de 1,1,1-trifluoroéthane,
. soumettre la phase liquide issue de la distillation précédente à une opération de chloration afin de chlorer les produits insaturés, tels que le chlorure de vinylidène restant dans le milieu et finalement,
. éliminer par distillation les produits chlorés formés à la phase précédente.

Cependant, s'il est aisé, quelle que soit la voie utilisée, d'éliminer le chlorure de vinylidène et le dichloracétylène, il n'en est pas nécessairement de même pour les composés insaturés chlorofluorés et plus particulièrement pour les 1,2-dichloro-1-fluoroéthylènes cis et trans nettement moins réactifs. Lorsque le traitement de chloration est réalisé dans des conditions plus sévères pour chlorer aussi ces composés insaturés chlorofluorés moins réactifs, une quantité assez importante de 1,1-dichloro-1-fluoroéthane peut réagir également, provoquant la formation de 1,1,2-trichloro-1-fluoroéthane. Cette perte de rendement en 1,1-dichloro-1-fluoroéthane a été observée dans tous les essais menés de manière plus sévère, tant par chloration photochimique qu'en présence d'un acide de Lewis.

De plus, l'élimination des 1,2-dichloro-1-fluoroéthylènes cis et trans apparaît d'autant plus difficile qu'il a été observé que, dans certains cas, après être passé par un minimum, leur concentration croît, si le traitement de chloration se prolonge. Une explication possible de ce phénomène serait leur formation par déshydrochloration du 1,1,2-trichloro-1-fluoroéthane formé au dépens du 1,1-dichloro-1-fluoroéthane. Cette explication ne lie cependant pas la demanderesse.

Par ailleurs, il a encore été observé que le 1,1-dichloro-1-fluoroéthane peut subir une dégradation importante en aval de l'étape de chloration des impuretés insaturées, notamment dans les étapes suivantes de distillation, lorsque l'on opère la chloration en présence de quantités égales ou supérieures à environ 10 ppm d'acides de Lewis ou lorsqu' il reste dans le milieu, après le réacteur de chloration, des quantités supérieures ou égales à environ 10 ppm d'acides de Lewis et notamment de FeCl₃. Le réacteur de chloration, ainsi que tous les appareillages en aval de celui-ci sont dès lors de préférence réalisés avec des matériaux résistant à la corrosion, tels que notamment le MONEL, l'INCONEL et l'HASTELLOY, non susceptibles d'introduire dans le milieu entre l'étape de chloration et la fin de la purification des quantités de FeCl₃ supérieures ou égales à 10 ppm.

Un mode préféré du procédé selon l'invention, consiste à remédier à ces inconvénients tout en rendant possible la transformation quasi complète de toutes les impuretés insaturées chlorées et chlorofluorées en chlorant ces impuretés pour former des produits saturés dont les points d'ébullition sont suffisamment différents de celui du 1,1-dichloro-1-fluoroéthane que pour pouvoir être ensuite aisément séparés par distillation.

Pour ce faire, la chloration est effectuée par mise en contact du milieu liquide à traiter avec du chlore en excès, en présence de très faibles quantités d'un acide de Lewis et de préférence de FeCl₃ à une température, ajustée en fonction de la quantité de FeCl₃, suffisamment élevée que pour permettre l'élimination quasi complète des impuretés en un temps raisonnable, ne dépassant pas quelques heures, mais suffisamment basse que pour éviter la chloration d'une partie du 1,1-dichloro-1-fluoroéthane. De manière particulièrement préférée, le 1,1-dichloro-1-fluoroéthane est débarrassé, préalabement à l'étape de chloration, des produits dont le point d'ébullition est supérieur à celui du 1,1-dichloro-1-fluoroéthane, c.à.d. essentiellement les oligomères et les goudrons. Cette séparation peut être réalisée classiquement par distillation.

La quantité de FeCl₃ mise en oeuvre, dans l'opération de chloration de ce mode préféré de l'invention, est obligatoirement inférieure à 10 ppm. De préférence, cette quantité est inférieure ou égale à environ 6 ppm. Bien que FeCl₃ soit mis en oeuvre en quantités très faibles, sa présence est cependant absolument indispensable pour réaliser la chloration de toutes les impuretés insaturées. La quantité minimale de FeCl₃ permettant une chloration des impuretés insaturées dans un délai raisonnable est environ de 0,05 ppm. De préférence, la quantité de FeCl₃ est supérieure ou égale à environ 1 ppm. De manière tout particulièrement préférée, elle est d'environ 3 ppm.

Vu les très faibles quantités de FeCl₃ utilisées, le procédé selon l'invention présente en outre l'avantage d'éviter une étape fastidieuse et délicate d'élimination du FeCl₃ du milieu par lavage alcalin ou par complexation, préalablement à toute opération ultérieure, étape absolument nécessaire pour éviter d'altérer la pureté du 1,1-dichloro-1-fluoroéthane lorsque la chloration est réalisée en présence de quantités plus importantes de FeCl₃.

En pratique, le FeCl₃ peut être introduit de différentes manières dans le chlorateur. Une façon ayant donné de bons résultats consiste en la saturation d'une fraction du flux de 1,1-dichloro-1-fluoroéthane à purifier par du FeCl₃. A titre indicatif, la solubilité du FeCl₃ dans le 1,1-dichloro-1-fluoroéthane à température ambiante est d'environ 7 ppm. D'autres solvants, dissolvant une quantité suffisante de FeCl₃, inertes dans les conditions de chloration et aisément récupérables dans une étape de séparation ultérieure, peuvent aussi être mis en oeuvre pour autant bien entendu qu'ils soient mis en oeuvre dans des quantités telles que la quantité totale de FeCl₃ présente dans le milieu réactionnel ne dépasse pas les valeurs citées ci-avant. Des solvants répondant à ces différents critères sont notamment le tétrachlorométhane, le trichlorométhane et le 1,2-dichloroéthane.

Avec les quantités de FeCl₃ définies ci-avant dans le milieu, une température d'environ 75 °C permet déjà d'effectuer la chloration des impuretés insaturées à une vitesse suffisante que pour en permettre une exploitation industrielle. Une bonne vitesse de chloration est obtenue lorsque la température est supérieure ou égale à environ 80 °C. Toutefois, à des températures supérieures à environ 120 °C, la transformation du 1,1-dichloro-1-fluoroéthane en 1,1,2-trichloro-1-fluoroéthane atteint des proportions importantes. De préférence, la chloration est réalisée à une température inférieure ou égale à environ 110 °C.

Le chlore est introduit en quantité excédentaire par rapport aux impuretés à chlorer. Le rapport molaire entre le chlore et la somme des impuretés insaturées à chlorer peut varier dans de larges mesures. Un rapport supérieur ou égal à environ 10 permet d'atteindre une vitesse de réaction acceptable; ce rapport peut aussi être beaucoup plus élevé sans pour cela provoquer la dégradation de 1,1-dichloro-1-fluoroéthane. Ce rapport ne dépasse en général pas 100.

La chloration peut être effectuée à pression atmosphérique ou à une pression supérieure à la pression atmosphérique. Cette pression peut être soit la pression autogène, soit une pression supérieure, générée par introduction d'un gaz inerte, par exemple, de l'azote. Habituellement, l'opération de chloration est effectuée à une pression d'environ 4 bar à environ 15 bar.

Dans les conditions définies ci-dessus, une chloration quasi complète des composés insaturés chlorés et chlorofluorés est obtenue avec un temps de traitement du 1,1-dichloro-1-fluoroéthane à purifier dans le chlorateur variant de quelques minutes à environ 2 heures. Industriellement, le temps de séjour du 1,1-dichloro-1-fluoroéthane dans le chlorateur est bien sûr ajusté en fonction de la pureté requise. Classiquement, une pureté satisfaisante est obtenue avec un temps de séjour de l'ordre de 10 à 60 minutes.

Les exemples 1 à 4 illustrent l'invention. L'exemple 5 est donné à titre de comparaison.

### Exemple 1

Dans un réacteur cylindrique (autoclave) de 0,5 litres en acier inoxydable 316, équipé d'un agitateur, refroidi à -30°C et mis sous vide de 15 mbars, on introduit le chlorure de vinylidène en premier sous agitation et on le laisse prendre la température du réacteur pendant quelques minutes de façon à obtenir une dépression dans l'autoclave.

Ensuite, le fluorure d'hydrogène, maintenu à température ambiante dans un cylindre en acier inoxydable 316, est introduit par aspiration dans le réacteur.

Le réacteur est ensuite plongé dans un thermostat préchauffé à la température voulue et la régulation de la pression est réglée à une valeur choisie, fonction de la température de l'essai, de façon à ne laisser échapper du réacteur que le chlorure d'hydrogène et les produits organiques plus légers que le 1,1-dichloro-1-fluoroéthane, c.à.d. essentiellement du 1-chloro-1,1-difluoroéthane et éventuellement le 1,1,1-trifluoroéthane.

L'instant auquel le réacteur est plongé dans le thermostat préchauffé détermine le début de la réaction.

Tout au long de la durée de la réaction, on suit l'évolution de la pression dans le réacteur tout en maintenant la température constante.

L'effluent gazeux est recueilli et mesuré dans un mesureur à eau après neutralisation et abattage de l'acide chlorhydrique et de l'acide fluorhydrique sortant dans un scrubber contenant une solution de NaOH diluée.

Enfin, après le temps de réaction voulu, le réacteur est brutalement refroidi à 0°C par un bain réfrigérant et les phases liquide et gazeuse sont sorties du réacteur (via un barboteur à NaOH relié au mesureur de gaz), et analysées.

Dans les conditions générales décrites ci-devant, on a réalisé trois essais en discontinu à des températures respectives de 75°C (essai 1), 100°C (essai 2) et 125°C (essai 3) dont les paramètres et les résultats sont repris ci-après.

| Essai 1 | | |
|---|---|---|
| Température de l'essai | | 75°C |
| Rapport molaire HF/VC₂ mis en oeuvre | | 2 mol/mol |
| Quantité de VC₂ mis en oeuvre | | 250 g |
| Quantité de HF mis en oeuvre | | 103 g |

| *Résultats en % mol après 5 h de réaction : | | |
|---|---|---|
| Taux de transformation du VC₂ | | 98,7 % |
| Sélectivité en 1,1-dichloro-1-fluoroéthane | | 88 % |
| Sous-produits : | . 1-chloro-1,1-difluoroéthane | 4 % |
| | . 1,1,1-trifluoroéthane | 3,3 % |
| | . total des oligomères | 4,7 % (exprimé en VC₂ transformé) |

| | | |
|---|---|---|
| * = ces résultats résultent d'une analyse par C.P.V. (chromatographie en phase gazeuse) reprenant la totalité des organiques présents dans la phase gazeuse et dans la phase liquide du réacteur | | |

| Essai 2 | | |
|---|---|---|
| Température de l'essai | | 100°C |
| Rapport molaire HF/VC₂ mis en oeuvre | | 2 mol/mol |
| Quantité de VC₂ mis en oeuvre | | 250 g |
| Quantité de HF mis en oeuvre | | 103 g |

| *Résultats en % mol après 2h30' de réaction : | | |
|---|---|---|
| Taux de transformation du VC₂ | | 98,7 % |
| Sélectivité en 1,1-dichloro-1-fluoroéthane | | 83,5 % |
| Sous-produits : | . 1-chloro-1,1-difluoroéthane | 10 % |
| | . 1,1,1-trifluoroéthane | 3 % |
| | . total des oligomères | 2,5 % (exprimé en VC₂ transformé) |

| | | |
|---|---|---|
| * = ces résultats résultent d'une analyse par C.P.V. (chromatographie en phase gazeuse) reprenant la totalité des organiques présents dans la phase gazeuse et dans la phase liquide du réacteur. | | |

| Essai 3 | | |
|---|---|---|
| Température de l'essai | | 125°C |
| Rapport molaire HF/VC₂ mis en oeuvre | | 2 mol/mol |
| Quantité de VC₂ mis en oeuvre | | 250 g |
| Quantité de HF mis en oeuvre | | 103 g |

| *Résultats en % mol après 3h30' de réaction : | | |
|---|---|---|
| Taux de transformation du VC₂ | | 99,3 % |
| Sélectivité en 1,1-dichloro-1-fluoroéthane | | 78 % |
| Sous-produits : | . 1-chloro-1,1-difluoroéthane | 16 % |
| | . 1,1,1-trifluoroéthane | 4 % |
| | . total des oligomères | 1 % (exprimé en VC₂ transformé) |

| | | |
|---|---|---|
| * = ces résultats résultent d'une analyse par C.P.V. (chromatographie en phase gazeuse) reprenant la totalité des organiques présents dans la phase gazeuse et dans la phase liquide du réacteur. | | |

On peut donc voir au départ des résultats en discontinu que l'hydrofluoration du VC₂ à haute température sans catalyseur(s) permet d'atteindre des taux de transformation du VC₂ élevés, avoisinant 100 % tout en observant des sélectivités en 1,1-dichloro-1-fluoroéthane situées vers 80 voire 90 %.

### Exemple 2

Dans un réacteur agité en acier inoxydable 316 de 200 cm³, à double enveloppe, chauffé à 120°C par circulation d'huile et débordant dans un tube de soutirage maintenu à la même température que le réacteur, on introduit en continu 52 g/h de HF et 138 g/h de VC₂, soit un rapport molaire à l'entrée du réacteur HF/VC₂ de 1,7 mol/mol.

La pression dans ce réacteur est régulée à 18 bars de façon à maintenir le milieu réactionnel liquide à 120°C.

Après 4 heures de mise en régime, on effectue des prélèvements de la phase gazeuse et de la phase liquide organique débordant de l'autoclave.

L'effluent, détendu à pression atmosphérique, est neutralisé dans un scrubber de lessive caustique diluée (1/10 molaire) et les gaz passent ensuite, via un système d'échantillonage composé de deux ampoules en pyrex de 100 cm³, dans un compteur humide permettant d'en mesurer le débit. On effectue également une extraction avec du tétrachlorure de carbone sur la phase aqueuse du scrubber afin d'obtenir une analyse complète de la totalité des organiques présents et formés dans le réacteur.

Les produits organiques sont ensuite analysés code à l'exemple 1 par chromatographie en phase vapeur, le HF non consommé est mesuré par ionométrie au moyen d'une électrode spécifique sensible aux ions F⁻ et le HCl formé est mesuré par potentiométrie au nitrate d'argent.

Les conditions opératoires et les résultats obtenus dans ces conditions sont repris au tableau I ci-dessous.

**TABLEAU I**

| | | |
|---|---|---|
| Température, | °C | 120 |
| Pression, | bars | 18 |
| Temps de séjour, | heures | 1 |
| Vitesse d'agitation, | tours/min. | 400 |
| Débit HF, | g/h | 52 |
| Débit VC2, | g/h | 138 |
| Rapport HF/VC2, | mol/mol | 1,7 |
| Taux de transformation VC2, | % | 94,1 |

| Sélectivités en : | | |
|---|---|---|
| . 1,1-dichloro-1-fluoroéthane, | % | 91,2 |
| . 1-chloro-1,1-difluoroéthane, | % | 6,3 |
| . 1,1,1-trifluoroéthane, | % | 0,6 |
| . 1,1,1-trichloroéthane, | % | 1,6 |
| . total des oligomères, | % | 0,2 (exprimé en VC2 transformé) |

On peut déduire des résultats de cet exemple 2 dans lequel la réaction d'hydrofluoration du VC₂ est conduite en continu, que les rendements et sélectivités peuvent encore être améliorés en comparaison avec les résultats excellents déjà observés selon l'exemple 1, où des expériences effectuées dans un procédé batch sont repris.

### Exemple 3

La phase organique d'une unité de préparation du 1,1-dichloro-1-fluoroéthane (teneur en 1,1-dichloro-1-fluoroéthane de 97 % poids), débarrassée par distillation des sous-produits plus volatils et plus lourds que le 1,1-dichloro-1-fluoroéthane, est introduite dans un réacteur en MONEL fonctionnant en continu. Simultanément, on introduit dans le réacteur du FeCl₃ en proportion telle que, dans le chlorateur, la phase organique renferme 0,6 ppm de FeCl₃ et 80 g de chlore par litre de phase organique.

Le réacteur de chloration est maintenu à une température de 100 °C ± 3 °C. La pression est ajustée à 9 bar au moyen d'azote. Le temps de séjour de la phase organique dans le réacteur est de 1 heure. Le tableau II reprend les teneurs initiales et finales en composés insaturés chlorés et chlorofluorés.

**TABLEAU II**

| | Teneur à l'entrée du chlorateur (ppm) | teneur à la sortie du chlorateur (ppm) |
|---|---|---|
| chlorure de vinylidène | 593 | < 5 |
| dichloracétylène | 173 | < 1 |
| 1,2-dichlorofluoroéthylène-cis | 77 | 8 |
| 1,2-dichlorofluoroéthylène-trans | 41 | < 5 |
| 1,2-dichloroéthylène-trans | 392 | < 5 |
| 1-chloro-1-fluoroéthylène | 5 | 2 |

### Exemple 4

La phase organique d'une unité de préparation du 1,1-dichloro-1-fluoroéthane (teneur en 1,1-dichloro-1-fluoroéthane de 97 % poids), débarrassée par distillation des sous-produits plus volatils et plus lourds que le 1,1-dichloro-1-fluoroéthane est introduite dans un réacteur en MONEL fonctionnant en continu. Sont aussi introduits dans le réacteur du FeCl₃ en proportion telle que, dans le chlorateur, la phase organique renferme 3 ppm de FeCl₃ et 80 g de chlore par litre de phase organique.

Le réacteur de chloration est maintenu à une température de 80 °C ± 3 °C. La pression est ajustée à 9 bar au moyen d'azote. Le temps de séjour de la phase organique dans le réacteur est de 45 minutes. Le tableau III reprend les teneurs initiales et finales des principaux composés insaturés chlorés et chlorofluorés.

**TABLEAU III**

| | Teneur à l'entrée du chlorateur (ppm) | teneur à la sortie du chlorateur (ppm) |
|---|---|---|
| chlorure de vinylidène | 431 | < 5 |
| dichloracétylène | 17 | < 1 |
| 1,2-dichlorofluoroéthylène-cis | 38 | < 5 |
| 1,2-dichlorofluoroéthylène-trans | 43 | < 5 |

### Exemple 5 (comparaison)

La phase organique d'une unité de préparation du 1,1-dichloro-1-fluoroéthane (teneur en 1,1-dichloro-1-fluoroéthane de 97 % poids), débarrassée par distillation des sous-produits plus volatils et plus lourds que le 1,1-dichloro-1-fluoroéthane est introduite dans un réacteur en MONEL fonctionnant en continu. Sont aussi introduits dans le réacteur du FeCl₃ en proportion telle que, dans le chlorateur, la phase organique renferme 40 ppm de FeCl₃ et 80 g de chlore par litre de phase organique.

Le réacteur de chloration est maintenu à une température de 80 °C ± 3 °C. La pression est ajustée à 9 bar au moyen d'azote. Le temps de séjour de la phase organique dans le réacteur est de 45 minutes. Le tableau IV reprend les teneurs initiales et finales des principaux composés insaturés chlorés et chlorofluorés.

**TABLEAU IV**

| | Teneur à l'entrée du chlorateur (ppm) | teneur à la sortie du chlorateur (ppm) |
|---|---|---|
| chlorure de vinylidène | 525 | < 5 |
| dichloracétylène | 28 | < 1 |
| 1,2-dichlorofluoroéthylène-cis | 41 | 20 |
| 1,2-dichlorofluoroéthylène-trans | 12 | 10 |

La comparaison des résultats décrits dans les Tableaux III et IV permet de constater l'influence extrêmement importante de faibles quantités de FeCl₃ (Tableau III) sur l'élimination des impuretés de type 1,2-dichlorofluoroéthylène cis et trans et dès lors par voie de conséquence sur la pureté du 1,1-dichloro-1-fluoroéthane finalement obtenu.

## Revendications

1. Procédé de préparation du 1,1-dichloro-1-fluoroéthane par hydrofluoration de chlorure de vinylidène caractérisé en ce que l'on effectue la réaction dans un milieu liquide à une température supérieure à 70°C en l'absence de catalyseurs.

2. Procédé selon la revendication 1 caractérisé en ce que la température du milieu liquide est comprise entre 75°C et 130°C.

3. Procédé selon la revendication 2 caractérisé en ce que la température est comprise entre 85°C et 125°C.

4. Procédé selon l'une quelconque des revendications 1 à 3 caractérisé en ce que la réaction est conduite sous pression autogène.

5. Procédé selon la revendication 4 caractérisé en ce que la pression est comprise entre 2 et 30 bars.

6. Procédé selon l'une quelconque des revendications 1 à 5 caractérisé en ce que le rapport molaire entre le fluorure d'hydrogène et le chlorure de vinylidène mis en oeuvre est compris entre 1,5 à 3 mol/mol.

7. Procédé selon l'une quelconque des revendications 1 à 6 caractérisé en ce que le chlorure de vinylidène résiduaire est bromé ou chloré.

8. Procédé de préparation de 1,1-dichloro-1-fluoroéthane avec une pureté supérieure à 99 % volume caractérisé en ce que l'on fait réagir à une température supérieure à 70°C du fluorure d'hydrogène et du chlorure de vinylidène en l'absence de tout catalyseur, en ce que l'on soumet la phase organique obtenue à une opération de distillation, en ce que la phase liquide issue de cette opération de distillation est soumise à une opération de chloration et en ce que l'on élimine les produits de chloration obtenus lors de cette opération de chloration par une autre opération de distillation.

9. Procédé selon la revendication 8 caractérisé en ce que l'opération de chloration est effectuée en présence de 0,05 à 6 ppm de FeCl₃, à une température de 75 à 120 °C en présence d'un excès de chlore.

10. Procédé selon les revendications 8 ou 9 caractérisé en ce que, préalablement à l'opération de chloration, la phase organique est soumise à deux opérations de distillation, l'une afin d'éliminer les produits dont le point d'ébullition est inférieur à celui du 1,1-dichloro-1-fluoroéthane, et l'autre afin d'éliminer les produits dont le point d'ébullition est supérieur à celui du 1,1-dichloro-1-fluoroéthane.

11. Procédé d'épuration du 1,1-dichloro-1-fluoroéthane en impuretés insaturées par chloration de ces impuretés au moyen de chlore introduit en quantité excédentaire par rapport aux impuretés à chlorer, caractérisé en ce qu'on effectue la chloration en présence de moins de 10 ppm de FeCl₃, à une température d'environ 75 à environ 120°C.

12. Procédé selon la revendication 11, dans lequel la chloration est effectuée en présence de 0,05 à 6 ppm de FeCl₃.

13. Procédé selon la revendication 11 ou 12, dans lequel la chloration est effectuée à une température d'environ 80 à environ 110°C.

14. Procédé selon l'une quelconque des revendications 10 à 13, dans lequel, préalablement à l'opération de chloration, le 1,1-dichloro-1-fluoroéthane à épurer est débarassé des produits dont le point d'ébullition est supérieur à celui du 1,1-dichloro-1-fluoroéthane.

## Claims

1. Process for the preparation of 1,1-dichloro-1-fluoroethane by hydrofluorination of vinylidene chloride, characterized in that the reaction is carried out in a liquid medium at a temperature above 70°C in the absence of catalysts.

2. Process according to Claim 1, characterized in that the temperature of the livid medium is between 75°C and 130°C.

3. Process according to Claim 2, characterized in that the temperature is between 85°C and 125°C.

4. Process according to any one of Claims 1 to 3, characterized in that the reaction is carried out under autogenous pressure.

5. Process according to Claim 4, characterized in that the pressure is between 2 and 30 bars

6. Process according to any one of Claims 1 to 5, characterized in that the molar ratio between hydrogen fluoride and vinylidene chloride used is between 1.5 and 3 mol/mol.

7. Process according to any one of Claims 1 to 6, characterized in that the residual vinylidene chloride is brominated or chlorinated.

8. Process for the preparation of 1,1-dichloro-1-fluoroethane having a purity of greater than 99% by volume, characterized in that hydrogen fluoride and vinylidene chloride are reacted at a temperature above 70°C in the absence of any catalyst, in that the organic phase obtained is subjected to a distillation operation, in that the liquid phase originating from this distillation operation is subjected to a chlorination operation and in that the chlorination products obtained during this chlorination operation are removed by another distillation operation.

9. Process according to Claim 8, characterized in that the chlorination operation is carried out in the presence of 0.05 to 6 ppm of FeCl₃ at a temperature of 75 to 120°C in the presence of an excess of chlorine.

10. Process according to Claim 8 or 9, characterized in that, prior to the chlorination operation, the organic phase is subjected to two distillation operations, one in order to remove the products whose boiling point is below that of 1,1-dichloro-1-fluoroethane, and the other in order to remove the products whose boiling point is above that of 1,1-dichloro-1-fluoroethane.

11. Process for the purification of 1,1-dichloro-1-fluoroethane from unsaturated impurities by chlorination of these impurities by means of chlorine introduced in an excess amount with respect to the impurities to be chlorinated, characterized in that the chlorination is carried out in the presence of less than 10 ppm of FeCl₃ at a temperature from approximately 75 to approximately 120°C.

12. Process according to Claim 11, in which the chlorination is carried out in the presence of 0.05 to 6 ppm of FeCl₃.

13. Process according to Claim 11 or 12, in which the chlorination is carried out at a temperature from approximately 80 to approximately 110°C.

14. Process according to any one of Claims 10 to 13, in which, prior to the chlorination operation, the 1,1-dichloro-1-fluoroethane to be purified is freed from products whose boiling point is above that of 1,1-dichloro-1-fluoroethane.

## Patentansprüche

1. Verfahren zur Herstellung von 1,1-Dichlor-1-fluorethan durch Hydrofluorierung von Vinylidenchlorid, dadurch gekennzeichnet, daß man die Reaktion in einem flüssigen Medium bei einer Temperatur höher als 70°C in Abwesenheit von Katalysatoren durchführt.

2. Verfahren nach Anspruch 1, dadurch gekennzeichnet, daß die Temperatur des flüssigen Mediums zwischen 75°C und 130°C liegt.

3. Verfahren nach Anspruch 2, dadurch gekennzeichnet, daß die Temperatur zwischen 85°C und 125°C liegt.

4. Verfahren nach einem der Ansprüche 1 bis 3, dadurch gekennzeichnet, daß die Reaktion unter autogenem Druck durchgeführt wird.

5. Verfahren nach Anspruch 4, dadurch gekennzeichnet, daß der Druck zwischen 2 und 30 Bar liegt.

6. Verfahren nach einem der Ansprüche 1 bis 5, dadurch gekennzeichnet, daß das molare Verhältnis zwischen dem Fluorwasserstoff und dem eingesetzten Vinylidenfluorid zwischen 1,5 und 3 Mol/Mol liegt.

7. Verfahren nach einem der Ansprüche 1 bis 6, dadurch gekennzeichnet, daß das restliche Vinylidenchlorid bromiert oder chloriert wird.

8. Verfahren zur Herstellung von 1,1-Dichlor-1-fluorethan mit einer Reinheit größer als 99 Vol.-%, dadurch gekennzeichnet, daß man bei einer Temperatur größer als 70°C Fluorwasserstoff und Vinylidenchlorid in Abwesenheit jedes Katalysators reagieren läßt, daß man die erhaltene organische Phase einem Destillierungsvorgang unterzieht, daß man die aus dieser Destillierungshase stammende flüssige Phase einem Chlorierungsvorgang unterzieht, und daß man die bei diesem Chlorierungsvorgang erhaltenen Chlorierungsprodukte durch einen anderen Destillierungsvorgang eliminiert.

9. Verfahren nach Anspruch 8, dadurch gekennzeichnet, daß der Chlorierungsvorgang in Anwesenheit von 0,05 bis 6 ppm FeCl₃, bei einer Temperatur von 75 bis 120°C in Anwesenheit eines Chlorüberschusses bewirkt wird.

10. Verfahren nach Anspruch 8 oder 9, dadurch gekennzeichnet, daß vor dem Chlorierungsvorgang die organische Phase zwei Destillierungsvorgängen unterzogen wird, einer zur Eliminierung der Produkte, deren Siedepunkt niedriger ist als der von 1,1-Dichlor-1-fluorethan, und der andere zur Eliminierung der Produkte, deren Siedepunkt höher ist als der von 1,1-Dichlor-1-fluorethan.

11. Verfahren zur Reinigung von 1,1-Dichlor-1-fluorethan von ungesättigten Verunreinigungen durch Chlorierung dieser Verunreinigungen mittels Chlor, das in einer bezüglich der zu chlorierenden Verunreinigungen überschüssigen Menge zugeführt wird, dadurch gekennzeichnet, daß man die Chlorierung in Anwesenheit von wenigstens 10 ppm FeCl₃ bei einer Temperatur von etwa 75 bis etwa 120°C durchführt.

12. Verfahren nach Anspruch 11, bei dem die Chlorierung in Anwesenheit von 0,05 bis 6 ppm FeCl₃ durchgeführt wird.

13. Verfahren nach Anspruch 11 oder 12, bei dem die Chlorierung bei einer Temperatur von etwa 80 bis etwa 110°C durchgeführt wird.

14. Verfahren nach einem der Ansprüche 10 bis 13, bei dem vor dem Chlorierungsvorgang das zu reinigende 1,1-Dichlor-1-fluorethan von Produkten befreit wird, deren Siedepunkt höher ist als der von 1,1-Dichlor-1-fluorethan.
